# EUROPÄISCHE PATENTSCHRIFT

(11) **EP 0 575 390 B1**
(45) Veröffentlichungstag und Bekanntmachung des Hinweises auf die Patenterteilung: **15.09.1999**
(21) Anmeldenummer: 92905948.3
(22) Anmeldetag: 07.03.1992
(51) Int. Cl.: A01H 5/00, C12N 5/04, A01H 4/00

(54) **GEGEN HERBIZIDE VOM ARYLOXY-PHENOXY-ALKANCARBONSÄURETYP RESISTENTER MAISZELLEN UND VERFAHREN ZUR HERSTELLUNG HERBIZIDRESISTENTER MAISZELLLINIEN**
MAIZE CELLS CAPABLE OF RESISTING HERBICIDES OF THE ARYLOXY-PHENOXY-ALKANE CARBOXYLIC ACID TYPE AND METHOD OF PRODUCING HERBICIDE-RESISTANT MAIZE CELL LINES
CELLULES DE MAIS RESISTANTES AUX HERBICIDES DU TYPE A L'ACIDE ARYLOXY-PHENOXYCARBOXYLIQUE D'ALCANE ET PROCEDE DE PRODUIRE DES CELLULES DE MAIS RESISTANTES AUX HERBICIDES

(30) Priorität: 12.03.1991 EP 91103765
(43) Veröffentlichungstag der Anmeldung: 29.12.1993
(60) Teilanmeldung: 98123134.3
(73) Patentinhaber: Hoechst Schering AgrEvo GmbH, 13342 Berlin (DE)
(72) Erfinder: DONN, Günter, D-6238 Hofheim (DE)
(86) Internationale Anmeldenummer: EP9200506
(87) Internationale Veröffentlichungsnummer: WO9216101

(56) Entgegenhaltungen:
- PROC.NATL.ACAD.SCI., USA Bd. 87, September 1990, Seiten 7175 - 7179; PARKER, W. B.,ET. AL: 'Dominant mutations causing alterations in acetyl-coenzyme A carboxylase confer tolerance to cyclohexanedione and aryloxyphenoxyprorionate herbicides in maize'
- PESTICIDE BIOCHEMISTRY PHYSIOLOGY Bd. 24, 1985, Seiten 298 - 305; HOPPE H H,ET.AL: 'Inhibition of fatty acid biosynthesis in isolated bean and maize chloroplasts by herbicidal phenoxy-phenoxypropionic acid derivatives and structurally related compounds'
- CHEMICAL ABSTRACTS, vol. 112, Columbus, Ohio, US; abstract no. 114108, GRONWALD, J.W., ET AL.: 'Selection for tolerance to graminicide hebicides in maize tissue culture'

## Beschreibung

Die Erfindung betrifft gegen Herbizide vom Aryloxy-Phenoxy-Alkancarbonsäuretyp resistente auxinautotrophe Maiszellen, -Protoplasten, Zellkuturen und Kalli, sowie ein Verfahren zur Herstellung herbizidresistenter Maiszelllinien.

Herbizide vom Aryloxy-Phenoxy-Alkancarbonsäuretyp (worunter hier auch Heteroaryloxy-Phenoxy-Alkancarbonsäure-Derivate verstanden werden sollen) sind wirksame Gräserherbizide. Stellvertretend für diese Wirkstoffklasse soll im folgenden das Fenoxaprop-ethyl ("FOPE") genannt werden, worunter sowohl das biologisch aktive D-Isomer als auch das Razemat zu verstehen ist. Sie wirken bei Pflanzen der Familie der Poaceae (Gramineae), da nur diese Pflanzenfamilie eine spezielle Form der Acetyl-CoenzymA-carboxylase (ACC) besitzen, die von FOPE in mikromolaren Konzentrationen inhibiert werden kann. Die übrigen Landpflanzen besitzen ACC-Typen, die um den Faktor 100 bis 1000 weniger empfindlich gegenüber dieser Wirkstoffklasse sind.

Da FOPE und andere Herbizide vom Aryloxy-Phenoxy-Alkancarbonsäuretyp über die oberirdischen Pflanzenteile aufgenommen wird, im Boden aber rasch inaktiviert werden, eignen sich diese Herbizide zur Gräserbekämpfung im Nachauflaufverfahren.

Die Kulturpflanze Mais (Zea mays) ist gegenüber FOPE besonders empfindlich. Dies macht den Einsatz dieser Verbindungen zur Bekämpfung von Schadgräsern in Maisfeldern unmöglich.

Es wurde gefunden, daß in Arealen, wo FOPE wiederholt angewendet wurde, in Wildgras-Populationen spontan Formen auftreten, die gegenüber dieser Herbizidklasse resistent sind. Da solche Mutationen nur in einer Rate von etwa 10⁻⁷ bis 10⁻⁹ auftreten, wäre eine entsprechende Suche nach Mutanten in Maisfeldern schon dann wenig aussichtsreich, wenn Mais die besonders hohe Empfindlichkeit gegen FOPE nicht aufwiese.

Unerwarteterweise wurde nun aber gefunden, daß Maiszellen selektiert werden können, die gegen FOPE resistent sind und die zu resistenten Pflanzen kultiviert werden können, die wiederum diese Resistenzeigenschaft stabil weitervererben.

Für die Selektion geeignete Zellinien sind bekannt (beispielsweise aus Morocz et al., Theor. Appl. Genet. 80 (1990) 721-726) bzw. in den Europäischen Patentanmeldungen 90 111 945.3 und 90 111 946.1 vorgeschlagen. Eine geeignete Zellinie wurde mit Wirkung vom 30.09.1990 bei der Deutschen Sammlung von Mikroorganismen und Zellkulturen unter dem Budapester Vertrag mit der Hinterlegungsnummer DSM 6009 hinterlegt.

Um resistente Zellinien zu erhalten, werden die Zellen in auxinfreien Medien in Anwesenheit von FOPE-Konzentrationen kultiviert, die mehr als 90 % der Zellen abtöten. In solchen Medien lassen sich die Zellen beliebig lange kultivieren, beispielsweise ohne weiteres über mehr als 10 Passagen. Synthetische Auxine wie 2,4-Dichlorphenoxyessigsäure, p-Chlorphenoxyessigsäure, 2,4,5-Trichlorphenoxyessigsäure und 3,6-Dichlor-2-methoxybenzoesäure (Dicamba) antagonisieren die Wirkung von FOPE, wenn dieses in subletalen Konzentrationen eingesetzt wird. Selektionsversuche mit FOPE im Konzentrationsbereich bis 10⁻⁵ M in Gegenwart synthetischer Auxine schlugen fehl. Es wurden deshalb auxinautotrophe Maiszellinien eingesetzt.

Auxinautotrophe Zellinien sind dadurch charakterisiert, daß sie beliebig lange, beispielsweise zwei bis drei Jahre, auf phytohormonfreien Zellkulturmedien als embryogene Kultur subkultiviert werden können. Auxinautotrophe Kalli wurden über mehr als 15 Passagen jeweils alle 3 bis 4 Wochen subkultiviert, und durch schrittweise Steigerung der FOPE-Konzentration konnten Mutanten gefunden werden. Diese FOPE-verträglichen Mutanten lassen sich auf FOPE-haltigem auxinfreiem Medium über mehr als 10 Passagen subkultivieren. Unter selektiven Bedingungen, d.h. in Anwesenheit von 10⁻⁴ M FOPE, entwickeln sich aus den embryogenen Kalli spontan Pflanzen, die sich zu fertilen Maispflanzen aufziehen lassen.

Blühende Regeneratpflanzen werden einerseits selbst bestäubt und andererseits Pollen der Regeneratpflanzen zur Bestäubung von Inzuchtlinien eingesetzt. Die reifen Samen werden ausgesät und die Sämlinge der F₁-Generation im 2 bis 4-Blatt-Stadium mit FOPE behandelt. Auch bei Konzentrationen bis zu 200 g Wirkstoff pro ha (g ai/ha) überlebt eine erhebliche Anzahl der Selektanten.

Die regenerierten Maispflanzen können mit bis zu 200 g ai/ha FOPE behandelt werden, vorzugsweise wird zwischen 20 und 150 g, insbesondere zwischen 30 und 90 g ai/ha, eingesetzt. Diese Mengenangaben gelten für das biologisch aktive D-Isomer des Fenoxaprop-ethyls. Erfindungsgemäße Maispflanzen werden vorzugsweise gegen das optisch aktive Isomer selektioniert, es können aber auch entsprechende Mengen des Razemats eingesetzt werden.

Aus den erfindungsgemäßen Mutanten kann in an sich bekannter Weise das ACC-Gen isoliert und charakterisiert werden. Mutierte Gene, die für eine FOPE-verträgliche ACC kodieren, können zur Transformation anderer Pflanzenzellen herangezogen werden.

Weiterhin ist es möglich, die FOPE-Verträglichkeit im Mais mit anderen Herbizid-Resistenzen zu kombinieren. Hierzu setzt man beispielsweise transgene Zellinien ein, die ein Resistenzgen gegen das nicht selektive Herbizid Phosphinothricin, Glufosinat oder Bialaphos enthalten. Derartige Gene sind beispielsweise aus den EP-A 0 257 542, 0 275 957, 0 297 618, aus der DE-A 3 701 623 oder der DE-B 3 825 507 bekannt. Bei der Anzucht entsprechender Zellinien kann die Phosphinothricin-Verträglichkeit als zusätzlicher Marker eingesetzt werden.

Andere erfindungsgemäße transgene Pflanzen können Toxingene, beispielsweise für das Bacillus thuringiensis δ-Endotoxin, oder Gene für Chitinasen oder Glucanasen sowie weitere selektierbare Markergene, beispielsweise Resistenzgene gegen Glyphosate oder Sulfonylharnstoffe enthalten.

Die folgenden Herbizide vom Typ der Aryloxyphenoxy-Alkancarbonsäure-(C₁-C₄)alkyl-, (C₂-C₄)alkenyl- und (C₃-C₄)alkinylester können ebenfalls zur Selektion resistenter Maiszellinien eingesetzt werden:
A1) Phenoxy-phenoxy- und Benzyloxy-phenoxy-Alkancarbonsäure-derivate z. B. 2-(4-(2,4-Dichlorphenoxy)-phenoxy)-propionsäuremethylester (Diclofop-methyl), 2-(4-(4-Brom-2-chlorphenoxy)-phenoxy)-propionsäuremethylester (s. DE-A-2601548),
   2-(4-(4-Brom-2-fluorphenoxy)-phenoxy)-propionsäuremethylester (s. US-A-4808750),
   2-(4-(2-Chlor-4-trifluormethylphenoxy)-phenoxy)-propionsäuremethylester (s. DE-A-2433067),
   2-(4-(2-Fluor-4-trifluormethylphenoxy)-phenoxy)-propionsäuremethylester (s. US-A-4808750),
   2-(4-(2,4-Dichlorbenzyl)-phenoxy)-propionsäuremethylester (s. DE-A-2417487), 4-(4-(4-Trifluormethylphenoxy)-phenoxy)-pent-2-en-säureethylester,
   2-(4-(4-Trifluormethylphenoxy)-phenoxy)-propionsäuremethylester (s. DE-A-2433067),
A2) "Einkernige" Heteroaryloxy-phenoxy-alkancarbonsäurederivate, z. B. 2-(4-(3,5-Dichlorpyridyl-2-oxy)-phenoxy)-propionsäureethylester (s. EP-A-2925),
   2-(4-(3,5-Dichlorpyridyl-2-oxy)-phenoxy)-propionsäurepropargylester (EP-A-3114),
   2-(4-(3-Chlor-5-trifluormethyl-2-pyridyloxy)-phenoxy-propionsäure-methylester (s. EP-A-3890),
   2-(4-(3-Chlor-5-trifluormethyl-2-pyridyloxy)-phenoxy)-propionsäure-ethylester (s. EP-A-3890),
   2-(4-(5-Chlor-3-fluor-2-pyridyloxy)-phenoxy)-propionsäurepropargylester (EP-A-191736),
   2-(4-(5-Trifluormethyl-2-pyridyloxy)-phenoxy-propionsäurebutylester Fluazifop-butyl (Fusilade-butyl),
A3) "Zweikernige" Heteroaryloxy-phenoxy-alkancarbonsäurederivate, z. B. 2-(4-(6-Chlor-2-chinoxalyloxy)-phenoxy)-propionsäuremethylester und -ethylester (Quizalofop-methyl und -ethyl),
   2-(4-(6-Fluor-2-chinoxalyloxy)-phenoxy)-propionsäuremethylester (s. J. Pest. Sci. Vol. 10, 61 (1985)),
   2-(4-(6-Chlor-2-chinolyloxy)-phenoxy)-propionsäuremethylester und -2-isopropylidenaminooxyethylester (Propaquizafop und Ester),
   2-(4-(6-Chlorbenzoxazol-2-yl-oxy)-phenoxy)-propionsäureethylester (Fenoxapropethyl) und
   2-(4-(6-Chlorbenzthiazol-2-yloxy)-phenoxypropionsäureethylester (s. DE-A-2640730)].

Die auxinautotrophen Zellinien eignen sich auch in besonderer Weise für die Selektion von Mutanten, die mit anderen Hemmstoffen der ACC, nämlich den Herbiziden vom Typ der Cyclohexandione, insbesondere Sethoxydim, Tralkoxydim. Cycloxydim, Alloxydim und Clethoxydim erhalten werden.

Aus der Veröffentlichung von Parker et al. (Proc. Natl. Acad. Sci. Vol. 87, pp. 7175-7179) ist bekannt, daß man Maispflanzen erhalten kann, die gegen übliche Konzentrationen von Sethoxydim resistent sind. Außerdem besitzen diese Pflanzen eine gewisse Kreuzresistenz gegen niedrige Konzentrationen von Haloxyfop. Die den erfindungsgemäß Zelllinien entstammenden Maispflanzen sind jedoch gegen höhere und für die praktische Anwendung notwendigen Konzentrationen von Aryloxyphenoxyalkancarbon-säureherbiziden resistent. Die den erfindungsgemäß Zelllinien entstammenden Maispflanzen gestatten somit die selektive Bekämpfung von monokotyledonen Schadpflanzen (Ungräsern) in Mais mittels Aryloxy - (einschließlich Heteroaryloxy)phenoxyalkancarbonsäurederivaten, entweder allein oder in Kombination miteinander.

Maispflanzen, die den erfindungsgemäß Zelllinien entstammen können mit einer Kombination von Aryloxyphenoxycarbonsäureherbiziden mit Herbiziden gegen dikotyledone Unkräuter behandelt werden. Es konnten nämlich überraschenderweise Kombinationspartner für die Aryloxyphenoxyalkancarbonsäure-herbizide identifiziert werden, die nicht nur von den regenerierten Maispflanzen toleriert werden, sondern deren herbizide Wirkung gleichzeitig verbessert wird.
Die Kombination der Herbizide verursacht also synergistische Effekte. Der Einsatz solcher Mischungen, ist mit großen wirtschaftlichen, aber auch ökologischen Vorteilen verbunden.

Unter Synergismus versteht man die sich gegenseitig verstärkende Wirkung von zwei oder auch mehr Stoffen. Im vorliegenden Fall bewirkt die kombinierte Anwendung von zwei Herbiziden, daß die Aufwandmenge der Herbizide reduziert werden kann und trotzdem die gleiche herbizide Wirkung erreicht wird, bzw. daß mit den gleichen Aufwandmengen der Herbizide eine höhere als die zu erwartende additive Wirkung der einzeln eingesetzten Wirkstoffe erzielt wird.

Durch Nutzung solcher synergistischer Effekte können die Aufwandmengen der beteiligten Mischungspartner erheblich reduziert werden und es gelingt ein breites Spektrum mono- und dikotyler Unkräuter in einem Arbeitsgang zu bekämpfen. Die Reduktion der Aufwandmengen betrifft insbesondere die ACC Hemmstoffe, aber auch die Kombinationspartner bezüglich der Wirksamkeit gegen dikotyle Unkräuter.

Von den Aryloxyphenoxyalkancarbonsäureherbiziden sind die folgenden Herbizide von besonderem Interesse: Fenoxaprop-ethyl, Haloxyfop-methyl, Quizalofop-ethyl, Fluazifop-butyl.

Als Mischungspartner zur zusätzlichen Bekämpfung von breitblättrigen Unkräutern zeigen folgende Herbizide synergistische Wirksamkeit:

Primisulfuron, Thifensulfuron, Nicosulfuron, DPX-E 9636, Amidosulfuron, Pyridylsulfonylharnstoffe, wie in den deutschen Patentanmeldungen P 4000503.8 und P 4030577.5 beschrieben, insbesondere 3-(4,6-Dimethoxypyrimidin-2-yl)-1-[3-(N-methyl-N-methylsulfonyl-amino-2-pyridyl-sulfonyl]-harnstoff, ein Alkoxyphenoxysulfonylharnstoff, wie in EP-A-0342569 beschrieben, ferner NC 319 (EP 282613) und andere Sulfonylharnstoffe sowie Mischungen verschiedener obenerwähnter Sulfonylharnstoffe untereinander wie z. B. eine Mischung aus Nicosulfuron und DPX-E 9636.

Auch andere Herbizide, die den gleichen oder ähnlichen Wirkungsmechanismus haben wie obige Sulfonylharnstoffe, nämlich Imidazolinone, wie z. B. Imazethapyr, Imazaquin, Imazapyr (die jeweils gemeinsam mit einem Safener in Mais eingesetzt werden können), zeigen synergistische Wirkungssteigerung, wenn sie zusammen mit ACC-Inhibitoren eingesetzt werden.

Ebenso kommen hier andere Herbizide in Betracht, die, wie Sulfonylharnstoffe und Imidazolinone, das Enzym Acetolactatsynthase (ALS) hemmen, z. B. substituierte Pyrimidine und Triazine, herbizide Sulfonamide, z. B. Flumetsulam (Cordes, R.C. et al., Abstr. Meet. Weed Sci. Soc. Am. 31, 10, 1991) oder andere verwandte Verbindungen und Mischungen solcher Wirkstoffe untereinander.

Eine Reihe weiterer Herbizide, die zur Bekämpfung von Unkräutern in Mais eingesetzt werden, aber andere Wirkungsmechanismen aufweisen, zeigten ebenfalls synergistische Wirkungssteigerung bei gemeinsamer Anwendung mit Fenoxaprop-ethyl bzw. anderen ACC-Inhibitoren:

ICI-051: (2-[2-chloro-4-(methyl-sulfonyl)benzoyl]-1,3-cyclohexandion, Atrazin, Cyanazin und Terbuthylazin, Clopyralid, Pyridate, Bromoxynil, Pendimethalin, Dicamba.

Die Aufwandmengen der Herbizide liegen im allgemeinen zwischen 0,01 und 2 kg/ha, d. h. die Gesamtmenge an aufzuwendender Wirkstoffkombination beträgt etwa 0,05 bis 2 kg/ha. Die erforderliche Aufwandmenge kann in Abhängigkeit von den äußeren Bedingungen wie Temperatur, Feuchtigkeit u. a. schwanken, vorzugsweise liegt sie zwischen 0,05 und 1 kg/ha. Die Mischungsverhältnisse können innerhalb weiter Grenzen schwanken. Vorzugsweise wird ein Mengenverhältnis zwischen 1:20 und 20:1 gewählt.

Diese synergistische Effekte werden sowohl bei Mischungen mit Fenoxaprop-ethyl wie auch bei Anwendung mit anderen ACC-Hemmstoffen, z. B. den Cyclohexandionen, erzielt. Eine Kombination der Wirkstoffe bedeutet, daß die herbiziden Wirkstoffe gemeinsam ausgebracht oder als sog. split-Applikation einige Tage nacheinander ausgebracht werden. In jedem Falle reagieren die Unkräuter mit erhöhter Sensibilität, so daß mit dem Einsatz geringerer Aufwandmengen eine sehr gute Bekämpfung erzielt werden kann.

In den folgenden Beispielen wird die Erfindung näher erläutert, ohne daß diese darauf beschränkt ist. Prozentangaben beziehen sich auf das Gewicht, sofern keine anderen Angaben gemacht sind.

### 1. Beispiel: Selektion von FOPE-vertraglichen embryogenen Maiszellkulturen

Maispflanzen der Inzuchtlinien B 73 und LH 82 wurden mit Pollen des regenerationsfähigen Genotyps HE 89 (Morocz et al., Theor. Appl. Genet. 80 (1990) 721-726 a.a.O.) bestäubt. 12 bis 14 Tage nach der Bestäubung wurden unreife Embryonen unter sterilen Bedingungen aus den Samen herauspräpariert und auf hormonfreien N₆-Kulturmedium (Chu et al., Sci. Sin. 18 (1975) 659-668), das 9 % Saccharose enthält, kultiviert, wobei die Embryoachse dem Medium aufliegt. An ca. 25 % der 1,0 bis 2,0 mm Embryonen bildete sich innerhalb von 3 bis 4 Wochen embryogener Kallus, der auf hormonfreiem Medium subkultivierbar war. Nach 3 bis 4 Subkulturen wurde mit den Kalluslinien, die sich durch gutes Wachstum und reproduzierbare Ausdifferenzierung von somatischen Embryonen auf dem hormonfreien Medium auszeichneten, begonnen, FOPE-verträgliche Mutanten zu selektieren.

Alternativ wurden Kalluslinien über 3 bis 4 Passagen auf N₆-Medium mit 1 mg/l 2,4-Dichlorphenoxyessigsäure (2,4 D) kultiviert. Zur Subkultur wurden die aus undifferenzierten Zellen aufgebauten Kallussektoren herangezogen. Aus diesen Kallussektoren ließen sich Suspensionskulturen induzieren, die in flüssigem N₆-Medium mit 0,5 mg/l 2,4 D kultiviert und wöchentlich in frisches Medium überführt wurden.

Sowohl von den Kalluskulturen als auch von den Suspensionskulturen wurde Gewebe entnommen und auf hormonfreiem N₆-Medium in Anwesenheit von 1-3 x 10⁻⁶ M FOPE 4 bis 6 Wochen inkubiert. Bis zu etwa 95 % der Zellen und Zellcluster starben unter diesen Bedingungen ab.

Die überlebenden Zellcluster wurden auf hormonfreiem N₆-Medium mit 3 x 10⁻⁶ M FOPE über 2 Passen weiter kultiviert. Pro Passage verblieben die Zellen 4 bis 6 Wochen auf dem Selektionsmedium.

Subklone, die unter diesen Bedingungen vergleichbar gut wuchsen wie Wildtypzellen auf FOPE-freiem Medium wurden auf hormonfreies N₆-Medium mit 1 x 10⁻⁵ M FOPE weiterkultiviert.

Nach weiteren 4 bis 6 Wochen wurden diejenigen Klone, die auf diesem Selektionsmedium ohne signifikante Wachstumsreduktion weiterwuchsen, auf ein Medium mit 3 x 10⁻⁵ M FOPE überführt und bei der folgenden Subkultur auf hormonfreies N₆-Medium mit 1 x 10⁻⁴ M Wirkstoff überführt. Höhere Wirkstoffkonzentrationen verstärkten den Selektionseffekt nicht weiter, da der Wirkstoff bereits bei 3 x 10⁻⁵ M im Medium auskristallisiert.

### Beispiel 2

### Regeneration von FOPE-verträglichen Pflanzen

Von denjenigen mutierten Klonen, die in Anwesenheit von 1 x 10⁻⁴ M FOPE in hormonfreiem N₆-Medium über 3 bis 10 Passagen ohne Wachstumsreduktion wuchsen und dabei aus somatischen Embryonen Pflanzen ausdifferenzierten, wurden die Regeneratpflanzen in Erde übergeführt und in einer Klimakammer bei 30 000 bis 40 000 Lux, 14 Stunden Beleuchtung, 23 ± 1°C Tagestemperatur und 16 ± 1°C Nachtemperatur aufgezogen. Sobald die Pflanzen 4 bis 5 Blätter entwickelt haben, werden sie mit 30 g FOPE pro ha besprüht. Die Pflanzen überlebten diese Behandlung ohne nennenswerte Schädigung, während Kontrollpflanzen von dieser Herbiziddosis abgetötet wurden.

Die blühenden Regeneratpflanzen wurden einerseits selbstbestäubt und andererseits wurde ihr Pollen dazu verwendet, Inzuchtlinien, wie beispielsweise B 73, LH 51, LH 82, LH 119, KW 1292, KW 5361, RA 1298 oder RA 3080, zu bestäuben. Die reifen Samen wurden ausgesät und die Sämlinge der F₁-Generation im 2 bis 4-Blatt-Stadium mit FOPE behandelt. Die Ergebnisse zeigt die Tabelle 1.

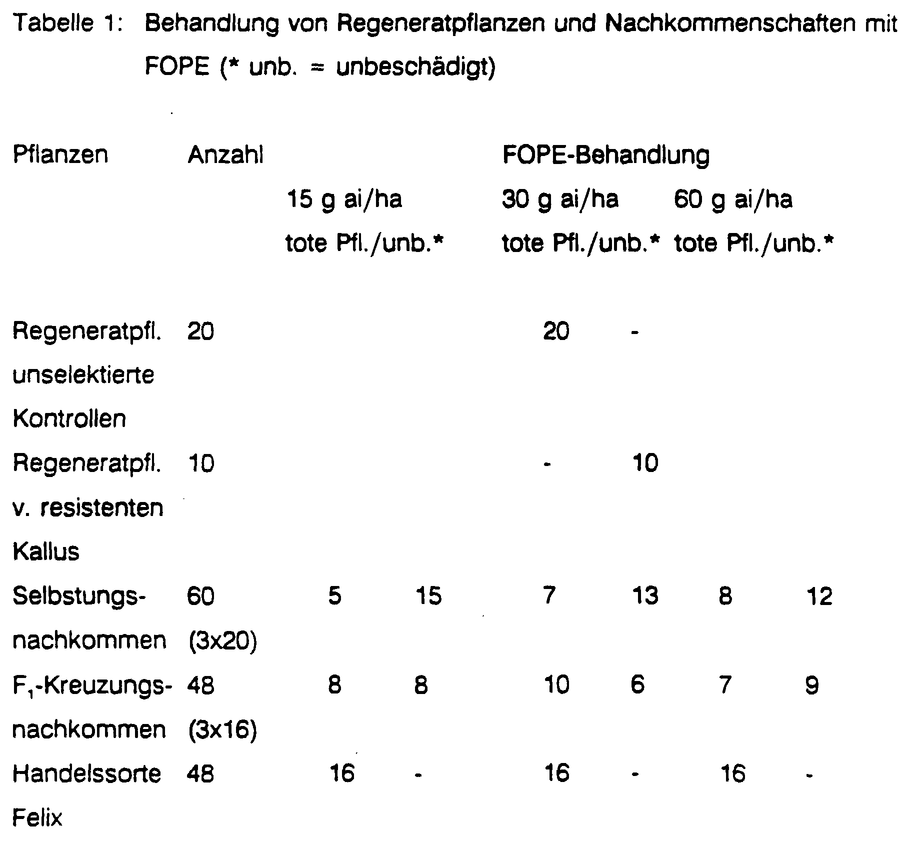

### 3. Beispiel: Haloxyfop-verträglicher Mais

Nach dem im 1. und 2. Beispiel beschriebenen Verfahren wurden resistente Maiszellinien erhalten und hinsichtlich der Resistenz gegen Haloxyfop untersucht. Dabei stellte sich heraus, daß die erfindungsgemäßen Zellinien eine deutlich höhere Dosis vertragen als die aus dem Stand der Technik bekannten Maiszellinien (siehe Parker et al.).

### 4. Beispiel: Behandlung herbizidresistenter Maispflanzen mit synergistischen Kombinationen von Herbiziden

Nach dem in dem 1. und 2.Beispiel beschriebenen Verfahren erhaltene FOPE-resistente Maispflanzen wurden gemeinsam mit grasartigen und breitblättrigen Unkräutern in Töpfen von 9 cm Durchmesser im Gewächshaus bis zum 4 - 6 Blattstadium herangezogen und mit den erfindungsgemäßen Herbiziden im Nachauflauf behandelt. Hierbei wurden ein Wasservolumen von 400 l/ha ausgebracht, 2 Wiederholungen durchgeführt und nach 5 Wochen Bonituren in Form von visuellen Schätzungen der Unkrautbekämpfung nach einer Prozentskala vorgenommen.

Die Ergebnisse der verschiedenen Versuche zeigten unerwartete synergistische Wirkungssteigerungen durch die gemeinsam oder kurz hintereinander ausgebrachten Herbizid-Kombinationen (siehe Tabelle 2 und 3).

An den herbizidresistenten Maispflanzen wurden keinerlei Schädigungen beobachtet. Die Herbizide B4 und B6 wurde jeweils gemeinsam mit einem Safenerwirkstoff appliziert.

**Tabelle 2**

| Herbizide Wirksamkeit gegen Gräser | | | | | | | |
|---|---|---|---|---|---|---|---|
| Herbizid | Dosis g AS/ha | % Wirkung gegen | | | | | |
| | | SEVI | DISA | PAMI | ECCG | SOHA | ZEMA |
| A | 50 | 100 | 100 | - | - | - | 0 |
| | 25 | 100 | 99 | 100 | 100 | 98 | 0 |
| | 12 | 85 | 85 | 100 | 100 | 85 | 0 |
| | 6 | 60 | 50 | 80 | 99 | 40 | 0 |
| B1 | 50 | 98 | 93 | 65 | 98 | 60 | 0 |
| | 25 | 95 | 85 | 20 | 95 | 35 | 0 |
| | 12 | 85 | 75 | 10 | 80 | 25 | 0 |
| B2 | 12 | 0 | 10 | 0 | 0 | 20 | 0 |
| | 6 | 0 | 5 | 0 | 0 | 0 | 0 |
| B3 | 50 | 50 | 20 | 30 | 30 | 90 | 0 |
| | 25 | 40 | 10 | 0 | 20 | 80 | 0 |
| | 12 | 30 | 0 | 0 | 20 | 70 | 0 |
| B4 | 25 | 80 | 90 | 65 | 95 | 45 | 0 |
| | 12 | 70 | 85 | 40 | 90 | 30 | 0 |
| | 6 | 60 | 70 | 15 | 75 | 20 | 0 |
| B5 | 25 | 85 | 95 | 70 | 90 | 70 | 0 |
| | 12 | 80 | 90 | 40 | 80 | 50 | 0 |
| | 6 | 80 | 80 | 25 | 70 | 30 | 0 |
| B6 | 100 | 95 | 90 | 70 | 70 | 60 | 0 |
| | 50 | 80 | 80 | 60 | 70 | 40 | 0 |
| B7 | 250 | 5 | 0 | 0 | 10 | 0 | 0 |
| | 125 | 0 | 0 | 0 | 0 | 0 | 0 |
| B8 | 250 | 0 | 0 | 0 | 0 | 0 | 0 |
| | 125 | 0 | 0 | 0 | 0 | 0 | 0 |
| B9 | 250 | 0 | 0 | 0 | 5 | 0 | 0 |
| | 125 | 0 | 0 | 0 | 0 | 0 | 0 |
| A + B1 | 12 + 12 | 100 | 100 | 100 | 100 | 98 | 0 |
| | 12 + 25 | 100 | 98 | 95 | 100 | 80 | 0 |
| A + B2 | 12 + 12 | 100 | 100 | 100 | 100 | 99 | 0 |
| | 6 + 12 | 100 | 99 | 100 | 100 | 75 | 0 |
| A + B3 | 12 + 12 | 100 | 100 | 100 | 100 | 100 | 0 |
| | 12 + 25 | 100 | 100 | 100 | 100 | 100 | 0 |
| A + B4 | 12 + 12 | 100 | 100 | 100 | 100 | 99 | 0 |
| | 6 + 6 | 100 | 95 | 95 | 100 | 90 | 0 |
| A + B5 | 12 + 12 | 100 | 100 | 100 | 100 | 100 | 0 |
| | 6 + 6 | 100 | 98 | 98 | 100 | 100 | 0 |
| A + B6 | 12 + 50 | 100 | 100 | 100 | 100 | 100 | 0 |
| | 6 + 25 | 100 | 99 | 98 | 100 | 95 | 0 |
| A + B7 | 12 + 125 | 95 | 90 | 100 | 100 | 95 | 0 |
| A + B8 | 12 + 125 | 90 | 95 | 100 | 99 | 90 | 0 |
| A + B9 | 12 + 125 | 95 | 90 | 99 | 99 | 90 | 0 |

**Tabelle 3**

| Herbizide Wirksamkeit gegen Breitblättrige | | | | | | | |
|---|---|---|---|---|---|---|---|
| Herbizid | Dosis g AS/ha | ABTH | CHAL | AMAR | POCO | AMRE | ZEMA |
| A | 50 | 0 | 0 | 0 | 0 | 5 | 0 |
| | 25 | 0 | 0 | 0 | 0 | 0 | 0 |
| | 12 | 0 | 0 | 0 | 0 | 0 | 0 |
| | 6 | 0 | 0 | 0 | 0 | 0 | 0 |
| B1 | 50 | 20 | 35 | 20 | 50 | 60 | 0 |
| | 25 | 10 | 20 | 10 | 30 | 50 | 0 |
| | 12 | 0 | 10 | 0 | 10 | 50 | 0 |
| B2 | 12 | 40 | 80 | 70 | 80 | 20 | 0 |
| | 6 | 20 | 60 | 40 | 50 | 10 | 0 |
| B3 | 50 | 70 | 80 | 30 | 40 | 40 | 0 |
| | 25 | 50 | 70 | 10 | 20 | 30 | 0 |
| | 12 | 40 | 50 | 0 | 20 | 15 | 0 |
| B4 | 25 | 20 | 95 | 85 | 80 | 80 | 0 |
| | 12 | 10 | 85 | 80 | 70 | 75 | 0 |
| | 6 | 0 | 50 | 70 | 70 | 60 | 0 |
| B5 | 25 | 30 | 80 | 75 | 30 | 60 | 0 |
| | 12 | 20 | 60 | 60 | 20 | 50 | 0 |
| | 6 | 10 | 40 | 40 | 10 | 30 | 0 |
| B6 | 100 | 60 | 40 | 70 | 50 | 80 | 0 |
| | 50 | 50 | 30 | 60 | 40 | 70 | 0 |
| B7 | 250 | 40 | 100 | 75 | 80 | 75 | 0 |
| | 125 | 15 | 85 | 40 | 50 | 30 | 0 |
| B8 | 250 | 70 | 95 | 70 | 85 | 65 | 0 |
| | 125 | 30 | 75 | 30 | 60 | 30 | 0 |
| B9 | 250 | 90 | 100 | 98 | 80 | 75 | 0 |
| | 125 | 75 | 80 | 70 | 40 | 60 | 0 |
| A + B1 | 12 + 12 | 40 | 50 | 30 | 60 | 90 | 0 |
| | 12 + 25 | 40 | 30 | 30 | 40 | 75 | 0 |
| A + B2 | 12 + 12 | 70 | 90 | 80 | 95 | 40 | 0 |
| | 6 + 12 | 60 | 80 | 75 | 90 | 40 | 0 |
| A + B3 | 12 + 12 | 60 | 70 | 30 | 50 | 30 | 0 |
| | 12 + 25 | 70 | 80 | 40 | 50 | 40 | 0 |
| A + B4 | 12 + 12 | 50 | 95 | 90 | 90 | 80 | 0 |
| | 6 + 6 | 30 | 60 | 80 | 90 | 75 | 0 |
| A + B5 | 12 + 12 | 50 | 90 | 95 | 40 | 90 | 0 |
| | 6 + 6 | 40 | 75 | 50 | 30 | 60 | 0 |
| A + B6 | 12 + 50 | 70 | 50 | 95 | 70 | 80 | 0 |
| | 6 + 25 | 50 | 30 | 60 | 40 | 60 | 0 |
| A + B7 | 12 + 125 | 40 | 90 | 50 | 70 | 50 | 0 |
| A + B8 | 12 + 125 | 60 | 80 | 40 | 60 | 40 | 0 |
| A + B9 | 12 + 125 | 90 | 90 | 80 | 50 | 60 | 0 |

### Legende zu Tabelle 2 und 3:

- SEVI: = Setaria viridis (Grüne Borstenhirse)
- DISA: = Digitaria sanguinalis (Bluthirse)
- PAMI: = Panicum miliaceum (Futterhirse)
- ECCG: = Echinochloa crus galli (Hühnerhirse)
- SOHA: = Sorghum halepense (Johnsongras)
- ABTH: = Abutilon theophrasti (Samtpappel)
- CHAL: = Chenopodium album (Weißer Gänsefuß)
- AMAR: = Ambrosia artemisifolia (Hohe Ambrosia)
- POCO: = Polygonum convolvulus (Windknöterich)
- AMRE: = Amaranthus retroflexus (Amaranth)
- ZEMA: = Zea mays (Mais)
- A: = Fenoxaprop-p-ethyl
- B1: = Nicosulfuron
- B2: = Thifensulfuron
- B3: = Primisulfuron
- B4: = 3-(4,6-Dimethoxypyrimidin-2-yl)-1-[3-(N-methyl-N-methylsulfonyl-amino)-2-pyridyl-sulfonyl]-harnstoff
- B5: = DPX-9636
- B6: = Imazethapyr
- B7: = Atrazin
- B8: = Bromoxynil
- B9: = Dicamba

## Patentansprüche

1. Gegen Aryloxyphenoxyalkancarbonsäureherbizide beständige auxinautotrophe Maiszellen, -Protoplasten, Zellkulturen und Kalli.

2. Maiszellen, Protoplasten, Zellkulturen und Kalli nach Anspruch 1, die eine Resistenz gegen ein weiteres Herbizid aufweisen.

3. Maiszellen, Protoplasten, Zellkulturen und Kalli nach Anspruch 1, die gegen Fenoxaprop-ethyl resistent sind.

4. Maiszellen, Protoplasten, Zellkulturen und Kalli nach Anspruch 1, die gegen 1 x 10⁻⁶ M bis 1 x 10⁻³ M Fenoxaprop-ethyl resistent sind.

5. Maiszellen, Protoplasten, Zellkulturen und Kalli nach Anspruch 1, die gegen 5 x 10⁻⁶ M bis 5 x 10⁻³ M Fenoxaprop-ethyl resistent sind.

6. Verfahren zur Herstellung herbizidresistenter Maiszellinien nach Anspruch 1, dadurch gekennzeichnet, daß man auxinautotrophe Maiszellinien schrittweise steigenden Konzentrationen von Aryloxyphenoxyalkancarbonsäureherbiziden aussetzt und die jeweils überlebenden Mutanten propagiert.

7. Verfahren nach Anspruch 6, dadurch gekennzeichnet, daß man auxinautotrophe Maiszellinien steigenden Konzentrationen zwischen 1 x 10⁻⁶ M und 1 x 10⁻³ M von Aryloxyphenoxyalkancarbonsäure-herbiziden aussetzt.

## Claims

1. An auxin-autotrophic maize cell, maize protoplast, maize cell culture or maize callus which is resistant to aryloxyphenoxyalkanecarboxylic acid herbicides.

2. A maize cell, maize protoplast, maize cell culture or maize callus as claimed in claim 1, which is resistant to a further herbicide.

3. A maize cell, maize protoplast, maize cell culture or maize callus as claimed in claim 1, which is resistant to fenoxaprop-ethyl.

4. A maize cell, maize protoplast, maize cell culture or maize callus as claimed in claim 1, which is resistant to 1 × 10⁻⁶ M to 1 × 10⁻³ M fenoxaprop-ethyl.

5. A maize cell, maize protoplast, maize cell culture or maize callus as claimed in claim 1, which is resistant to 5 × 10⁻⁶ M to 5 × 10⁻³ M fenoxaprop-ethyl.

6. A method for the production of herbicide-resistant maize cell lines as claimed in claim 1, which comprises exposing auxin-autotrophic maize cell lines stepwise to increasing concentrations of aryloxyphenoxyalkanecarboxylic acid herbicides and propagating the mutants which survive in each case.

7. The method as claimed in claim 6, wherein auxinautotrophic maize cell lines are exposed to aryloxyphenoxyalkanecarboxylic acid herbicides at increasing concentrations of between 1 × 10⁻⁶ M and 1 × 10⁻³ M.

## Revendications

1. Cellules, protoplastes de maïs, cultures cellulaires et cals, autotrophes pour la production d'auxines, résistants aux herbicides aryloxyphénoxyalcanecarboxyliques.

2. Cellules de maïs, protoplastes, cultures cellulaires et cals selon la revendication 1, qui présentent une résistance contre un autre herbicide.

3. Cellules de maïs, protoplastes, cultures cellulaires et cals selon la revendication 1, qui sont résistants contre le fénoxapropéthyl.

4. Cellules de maïs, protoplastes, cultures cellulaires et cals selon la revendication 1 qui sont résistants contre le fénoxapropéthyl de 1 x 10⁻⁶ M jusqu'à 1 x 10⁻³ M.

5. Cellules de maïs, protoplastes, cultures cellulaires et cals selon la revendication 1 qui sont résistants contre le fénoxapropéthyl de 5 x 10⁻⁶ M jusqu'à 5 x 10⁻³ M.

6. Procédé pour la préparation de lignées cellulaires de maïs selon la revendication 1 résistantes aux herbicides, caractérisé en ce qu'on expose progressivement des lignées cellulaires de maïs autotrophes pour la production d'auxines à des concentrations croissantes d'herbicides aryloxyphénoxyalcanecarboxyliques et en ce qu'on propage les mutants respectivement survivants.

7. Procédé selon la revendication 6, caractérisé en ce qu'on expose progressivement des lignées cellulaires de maïs autotrophes pour la production d'auxines à des concentrations croissantes entre 1 x 10⁻⁶ M et 1 x 10⁻³ M d'herbicides aryloxyphénoxyalcanecarboxyliques.
